# EUROPEAN PATENT SPECIFICATION

(11) **EP 0 923 359 B1**
(45) Date of publication and mention of the grant of the patent: **19.11.2003**
(21) Application number: 96921060.8
(22) Date of filing: 04.07.1996
(51) Int. Cl.: A61F 11/00, A61B 17/24

(54) **EAR AND NOSE HYGIENE DEVICE**
OHREN- UND NASENREINIGER
DISPOSITIF POUR L'HYGIENE DU NEZ ET DES OREILLES

(43) Date of publication of application: 23.06.1999
(73) Proprietor: Begun, Jacob, Mazkeret Batya 76804 (IL); Giniger, Arie, Kfar Shmariyahu 46910 (IL)
(72) Inventor: Begun, Jacob, Mazkeret Batya 76804 (IL); Giniger, Arie, Kfar Shmariyahu 46910 (IL)
(74) Representative: Thomson, Paul Anthony
(86) International application number: IL9600035
(87) International publication number: WO98001096

(56) References cited:
- EP-A- 0 744 168
- WO-A-96/14033
- DE-C- 857 128
- DE-U- 9 308 737
- FR-A- 2 551 657
- GB-A- 2 204 496
- US-A- 1 658 801
- US-A- 2 569 237
- US-A- 4 981 143
- US-A- 5 348 023

## Description

### FIELD OF THE INVENTION

The present invention relates to a personal hygiene device. More specifically it concerns a device for cleaning the ear canal and/or for cleaning the nostrils.

### BACKGROUND OF THE INVENTION

Cleaning of ear wax or scratching the ear canal in response to itching may be performed by "*improvise*" implements such as toothpicks, soiled handkerchiefs, etc., or by means of a cotton swab. The use of improvised implements may be damaging and further may result in a driving of accumulated wax further down the ear canal. Use of a swab may result in an undesirable drying of the ear canal.

Cleaning of nostrils, particularly of infants, is necessary at times to allow free breathing. It may be achieved by similar devices, or with similar associated problems. In addition, in the case of infants, it is important to ensure free breathing also while cleaning.

Various types of such cleaning devices are known. U.S. Patent No. 147,660 teaches the use of an ear cleaner consisting of a handle made of a twisted wire, furnished at one or both ends with a loop for the attachment of a swab, with or without a spoon at the opposite side of the cleaner. U.S. Patent No. 2,096,162 discloses an implement for cleaning the nostrils which is comprised of a stem and an end portion in the form of a clover leaf. German Patent DE-3,633,585 describes a device which is comprised of a shaft of flexible material attached to a knob. The shaft has a helical ridge extending over its whole length and terminating in a flat surface. U.S. Patent 3,203,418 discloses an ear swab having a disposable swab member securely attached thereon which can be replaced by selectively removing it therefrom. U.S. Patent 5,334,212 discloses an apparatus which comprises a loop curette to extract wax from the ear and a safety stopper having a slit and central resting hole to control the insertion depth of the loop curette.

FR 2,551,657 describes a personal hygiene device with a solid spoon-like broad end body at either end of a stem for gathering exudate. WO 96/14033 describes a personal hygiene device with a stem having a ring shaped head coated with a sensitive material. US 1,658,801 describes a personal hygiene device with a broad-end body having recessed cavities for gathering exudate. US 4,981,143 describes a cell sampler for obtaining specimens and having a stick-shaped handle with a stick-shaped abrading segment for scraping. purposes. US 2,569,237 describes an ear cleaning instrument with an elongated loop portion for gathering exudate. DE-U-93 08 737.3 describes an ear cleaning instrument with a cork-screw like end for removing exudate. GB 2 204 496 A describes a device having a shaft with a loop-like collection means at one end for collecting biological material, the collection means having a transverse cross section of similar dimension to the shaft.

These known devices and others, although allowing some degree of cleaning of the ear canal or the nostrils do not perform sufficiently well, i.e. effective cleaning on the one hand, and performing this art in order to avoid hurting the ear canal and the tympanic membrane during ear cleaning or hurting the delicate membranes on the inner surface of the nostril.

### SUMMARY OF THE INVENTION

It is an object of the present invention to provide a device which can be used efficiently for cleaning and/or scratching of the ear or the nostrils.

Other objects of the invention will become apparent as the description proceeds.

In the following description the term "*exudate*" will be used to denote both ear wax and nasal mucus.

In accordance with the present invention, a personal hygiene device adapted for insertion into the ear canal or the nostrils for removing exudate from an exudate-containing ear canal or nostril, the personal hygiene device being an oar-like device comprising a stem having a longitudinal axis and a broad end body at one end thereof, said broad end body having a rim portion having the form of a loop surrounding one or more exudate engaging members integrally formed therewith,
characterized in
said engaging members being a plurality of projections extending inwardly and transversely toward said longitudinal axis.

In accordance with a preferred embodiment of the invention, said rim portion has a smooth external surface so as to allow minimal friction between the device and the surrounding walls of the ear canal or the nostrils.

According to one embodiment of the invention, said rim portion has the form of a loop with said engaging members being a plurality of inwardly directed projections. Although not part of the present invention, for information only, it is appreciated that engaging members can be a grid or a perforated element. However, a plurality of partitions extending between opposite sections of the rim portion is considered to form an engaging member of the present invention.

Said body may be planar, or it may be bent or twisted so as to increase the surface of the area which comes into contact with the exudate to be removed and/or to be adapted to fit the space within the ear canal or nostril. The broad end portion may have a variety of other desired forms, e.g. it may be convex, concave, concavo-convex, etc.

According to an embodiment of the invention, the device comprises a safety stopper, which defines a maximum insertion depth of said. device. This may be important particularly for such devices intended for use in children or for use by a nurse within the framework of treatment of disabled individuals.

The device is typically made out of plastic, but as will no doubt be appreciated by the artisan, the device may also be made from a number of other materials such as wood, metal, ceramic material, etc.

The invention will now be illustrated by a description of some specific embodiments. In the described embodiments, the device comprises a single broad end body and it will be appreciated that the invention is not limited thereto and encompasses in its scope also such devices comprising two end bodies, each at an end of said stem. It will further be appreciated, that the described embodiments are by way of example only and the invention is not limited to these embodiments.

### BRIEF DESCRIPTION OF THE DRAWINGS

**Figs. 1-3** are front views of a device in accordance with the invention where the exudate engaging members are inwardly extending projections;
**Figs. 4** and **5** are front views of devices, shown for information purposes only, where the exudate engaging member is a perforated matrix;
**Fig. 6** is a front view of the device in accordance with the embodiment of the invention where the exudate engaging members are parallel partitions;
**Fig. 7** shows a device, for information purposes only, where the exudate engaging member has the form of a grid;
**Fig. 8** shows a device in accordance with an embodiment of the invention where the broad end portion is bent with respect to the longitudinal axis of the stem; and
**Fig. 9** shows a device in accordance with an embodiment of the invention provided with a safety stopper:
**Fig. 9a** shows a front view of the device;
**Fig. 9b** shows a top view of the device.

### DESCRIPTION OF SPECIFIC EMBODIMENTS

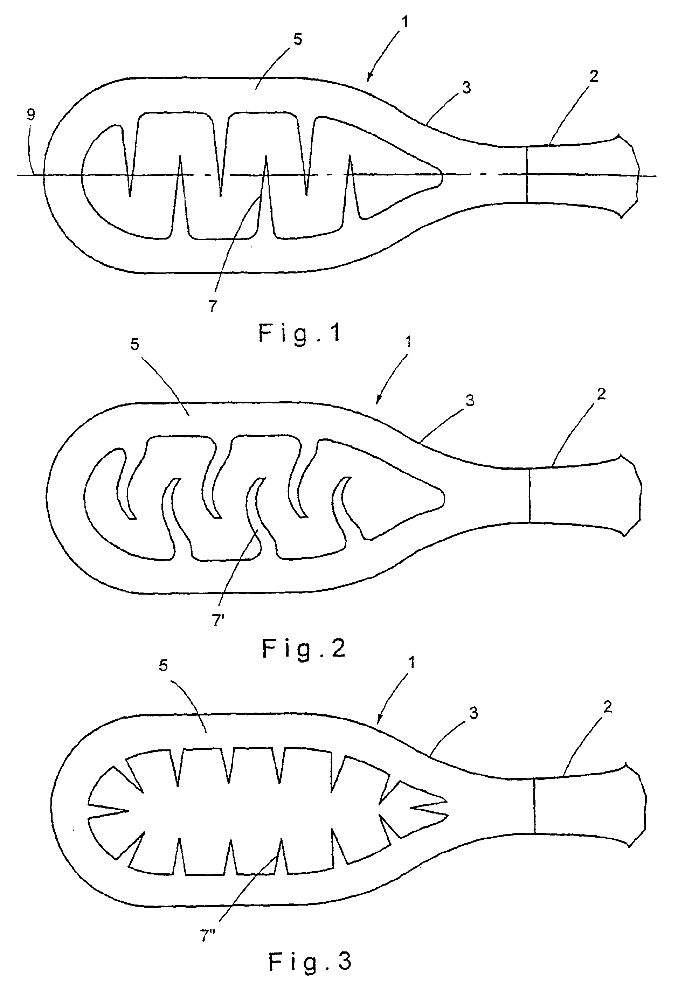

Reference is first being made to Fig. 1 showing a device in accordance with the embodiment of the invention, generally designated **1** comprising a stem **2** and a broad end portion **3**. The stem portion **2**, only a small part of which is shown, is typically several times the length of the broad end portion **3**.

Broad end portion **3** has a rim portion **5** being provided with a plurality (6 in this embodiment) of inwardly directed projections **7**. These projections in this embodiment (unlike the embodiment in Fig. 3 - see below) extend beyond the longitudinal axis **9** of the device.

The embodiments shown in Figs. 2 and 3 are similar to that of Fig. 1. and like elements were given like numbers. The difference in these embodiments lies in the structure of projections **7'** and **7"** as compared to projections **7** of the embodiment in Fig. 1. In Fig. 2, projections **7'** have somewhat wavy and curved, structure, whereas as projection **7"** have a similar geometry to projection **7** of the embodiment of Fig. 1 but being shorter and not crossing the longitudinal axis of the device.

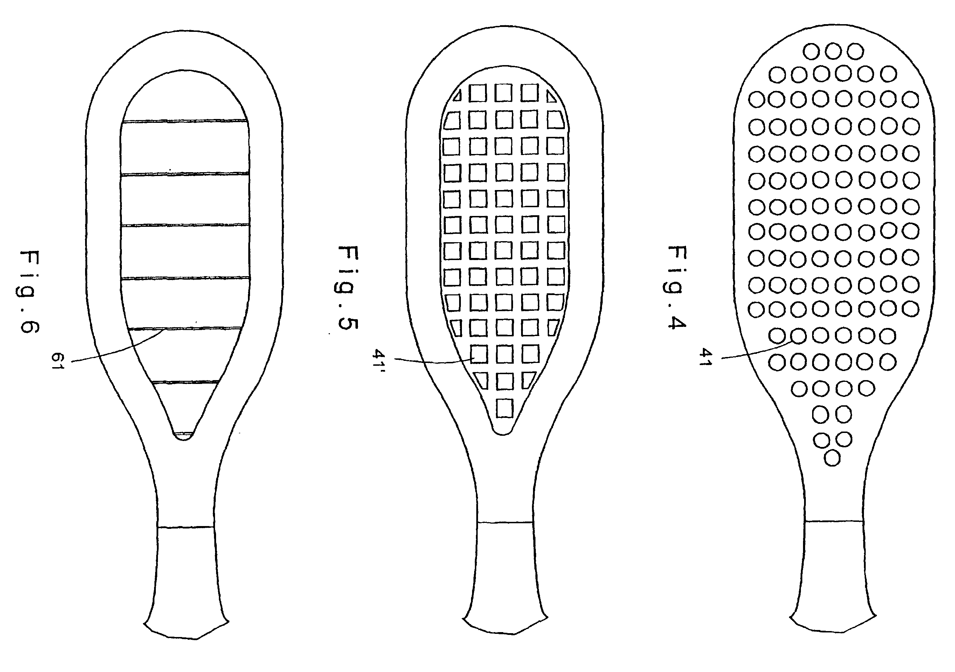

In the device according to Fig. 4, the central portion, which is the exudate engaging member, is perforated, with the perforation providing larger surface for engagement with the exudate. The device of Fig. 5 is essentially similar to that of Fig. 4, with the main difference residing in the structure of the exudate engagement member 41', in that the perforations have a rectangular cross-section.

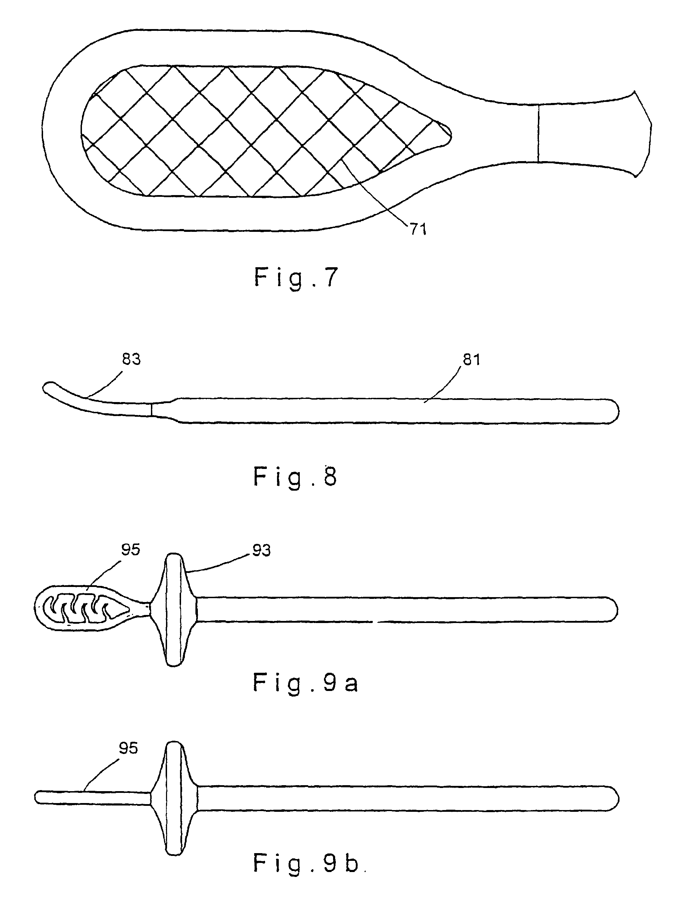

Fig. 6 shows a device in accordance with an embodiment of the invention comprising a plurality of exudate engaging members which have the form of parallel partitions **61**. In the device of Fig. 7, the engaging member has the form of grid **71**.

The device of the embodiment shown in Fig. 8 has a stem **81** and a broad end portion **83** with a curved profile. It will be appreciated that in addition to a curved profile;-the broad end portion may have a variety of other desired forms, e.g. it may be twisted, convex, concave, concavo-convex, etc.

The device of the invention may also be equipped, as shown in Fig. 9, with a safety stopper **93**. Such a stopper is intended to control the depth of penetration into the ear canal or nostril of the broad end body **95**.

The two components of the device, namely the stem and the broad end portion, may be integral with one another, or may be two separate members which are adapted to be engaged with one another.

## Claims

1. A personal hygiene device (1) adapted for insertion into the ear canal or the nostrils for removing exudate from an exudate containing ear canal or nostril, the personal hygiene device being an oar-like device comprising a stem(2) having a longitudinal axis and a broad end body (3) at one end thereof, said broad end body having a rim portion (5) having the form of a loop and surrounding one or more exudate engaging members (3) integrally formed therewith,
**characterized in**
said engaging members (7) being a plurality of projections extending inwardly and transversely toward said longitudinal axis.

2. The personal hygiene device according to Claim 1 wherein said at least one exudate engaging member is constituted by a plurality of partitions each extending across said rim portion and transversing said longitudinal axis.

3. The personal hygiene device according to either Claim 1 or 2 wherein said broad end body (3) is convex in a side view thereof

4. The personal hygiene device according to any one of Claims 1-3 wherein said broad end body (3) is twisted.

5. The personal hygiene device according to any one of Claims 1-4 and further comprising a safety stopper (93) for defining a maximum insertion depth of said device.

## Patentansprüche

1. Ein Körperpflegeartikel (1 ), ausgebildet, um in einen Ohrkanal oder in ein Nasenloch zum Entfernen von Exsudat aus einem Exsudat enthaltenen Ohrkanal oder einen Nasenloch eingeführt zu werden, wobei der Körperpflegeartikel nach Art eines Ruders ausgebildet ist und einem Schaft (2) mit einer Längsachse und mit an einem Ende einen breiten Endkörper (3) desselben umfasst, wobei der breite Endkörper einen Randabschnitt (5) in Form einer Schlinge und ein oder mehrere Exsudat aufnehmende Glieder (7) als integralen Bestandteil derselben aufweist, **dadurch gekennzeichnet, dass** es sich bei den Aufnahmegliedern (7) um eine Vielzahl von sich nach innen und quer zur Längsachse erstreckenden Fortsätze handelt.

2. Körperpflegeartikel nach Anspruch 1, bei dem das wenigstens eine Exsudat aufnehmende Glied durch eine Vielzahl von Teilungen gebildet ist, die sich jeweils über den Randabschnitt erstrecken und die Längsachse übergreifen.

3. Körperpflegeartikel nach Anspruch 1 oder 2, bei dem der weite Endkörper (3) in einer Seitenansicht konvex ist.

4. Körperpflegeartikel nach einem der Ansprüche 1 bis 3, bei dem der weite Endkörper (3) verdreht ist.

5. Körperpflegeartikel nach einem der Ansprüche 1 bis 4, der ferner ein Sicherheitsstoppelement (93) umfasst, durch das eine maximale Einsetztiefe des Artikels definiert ist.

## Revendications

1. Dispositif (1) pour l'hygiène personnelle convenant à l'insertion dans le canal auditif ou dans les narines pour enlever l'exsudat d'un canal auditif ou d'une narine contenant un exsudat, le dispositif d'hygiène personnel étant un dispositif adoptant la forme d'un aviron, comprenant une tige (2) ayant un axe longitudinal et un corps (3) à extrémité large à une extrémité de celle-ci, ledit corps à extrémité large ayant une partie de bordure (5) ayant la forme d'une boucle et entourant un ou plusieurs éléments (7) d'entraînement de l'exsudat formés solidaires de celle-ci,
***caractérisé en ce que***
lesdits éléments d'entraînement (7) sont une pluralité de prolongements s'étendant intérieurement et transversalement en direction dudit axe longitudinal.

2. Dispositif pour l'hygiène personnelle selon la revendication 1 dans lequel ledit au moins un élément d'entraînement de l'exsudat est constitué d'une pluralité de parois dont chacune s'étend d'un côté à l'autre de la partie de bordure et transversalement audit axe longitudinal.

3. Dispositif pour l'hygiène personnelle selon la revendication 1 ou 2 dans lequel ledit corps (3) à extrémité large est convexe selon une vue latérale de celui-ci.

4. Dispositif pour l'hygiène personnelle selon l'une quelconque des revendications 1 à 3 dans lequel ledit corps (3) à extrémité large est torsadé.

5. Dispositif pour l'hygiène personnelle selon l'une quelconque des revendications 1 à 4 et comprenant de plus un butoir de sécurité (93) destiné à définir une profondeur maximale d'insertion dudit dispositif.
